# EUROPEAN PATENT SPECIFICATION

(11) **EP 2 079 436 B1**
(45) Date of publication and mention of the grant of the patent: **10.11.2010**
(21) Application number: 07800663.2
(22) Date of filing: 26.09.2007
(51) Int. Cl.: A61K 8/40, C07C 255/07, C07C 255/31, C11B 9/00, A61Q 13/00

(54) **ALPHA, BETA, UNSATURATED NITRILES AS FRAGRANCES**
ALPHA-, BETA-UNGESÄTTIGTE NITRILE ALS DUFTSTOFFE
NITRILES ALPHA,BETA-INSATURES UTILISES COMME PARFUMS

(30) Priority: 26.09.2006 GB 0618870
(43) Date of publication of application: 22.07.2009
(73) Proprietor: Givaudan SA, 1214 Vernier (CH)
(72) Inventor: GRANIER, Thierry, 8600 Duebendorf (CH); GAILLARD, Antoine, 01220 Divonne (FR)
(74) Representative: Sievert, Claudia
(86) International application number: PCT/CH2007/000472
(87) International publication number: WO 2008/037105

(56) References cited:
- EP-A- 0 395 982
- EP-A- 0 419 980
- WO-A-2006/066437
- DE-A1- 10 022 076
- US-A- 4 451 479
- US-A- 6 114 565
- DATABASE WPI Week 199941 Derwent Publications Ltd., London, GB; AN 1996-300542 XP002459012 & ES 2 133 179 T3 (QUEST INT) 1 September 1999 (1999-09-01)

## Description

The present invention refers to the use of short chain alk-2-enenitriles as perfuming ingredients and to flavour and fragrance compositions comprising them.

In the fragrance industry there is a constant demand for new compounds that enhance or improve on odour notes.

Short chain alk-2-enenitriles are known from the prior art. However, to the best of our knowledge, the prior art is completely silent with regard to their organoleptic properties or any use of this class of compounds in the field of perfumery.

The only short chain nitriles suitable in the field of perfumery described in literature are 3-butenenitrile and 4-pentenenitrile possessing onion-like and pungent mustard-like odour notes respectively (6th Int. Congr. Essent. Oils 1974, 73, W. S. Brud et al.).

Surprisingly, inventors found that certain short chain alk-2-enenitriles possess very interesting green, fruity odour notes.

Accordingly, the present invention refers in one of its aspects to the use as fragrance or flavour ingredient of a compound of formula (I) 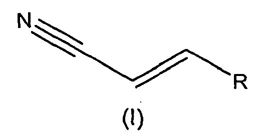 wherein R is selected from linear, branched C₃₋₄ alkyl and C₃₋₄cycloalkyl, such as propyl, prop-2-yl, butyl, 1-methylprop-1-yl, 2-methylprop-1-yl and cyclopropyl; and the double bond is in E or Z configuration; or a mixture thereof.

Whereas the (Z)- and (E)-isomers show similar olfactory profiles, the odour threshold of the (Z)-isomer compared to its corresponding (E)-isomer is higher. Accordingly, the use of the pure (E)-isomer is preferred. However, a mixture of both isomers, preferably enriched in the (E)-isomer, which is readily available synthetically, can also be used. The term "enriched" is used herein to describe the compounds of the present invention having an isomeric purity greater than 1:1 in favour of the (E)-isomer. Particularly preferred are compounds having an isomeric purity of 1.5:1 (E:Z), more preferably 2:1 (E:Z) or 3:1 (E:Z), and most preferably 5:1 (E:Z) or greater.

The compounds according to the present invention may contain one stereocenter, and as such exist as mixtures of stereoisomers. They can be used as stereoisomeric mixtures, or may be resolved in diastereomerically and/or enantiomerically pure form. Resolving stereoisomers adds to the complexity of manufacture and purification of these compounds, and so it is preferred to use the compounds as mixtures of their stereoisomers simply for economic reasons. However, if it is desired to prepare individual stereoisomers, this may be achieved according to methodology known in the art, e. g. preparative HPLC and GC or by stereoselective syntheses.

In particular embodiments are the use as flavour or fragrance ingredient of compounds of formula (I) selected from the list consisting of hex-2-enenitrile, hept-2-enenitrile, 4-methylhex-2-enenitrile, 5-methylhex-2-enenitrile, 4-methylpent-2-enenitrile and 3-cyclopropylacrylonitrile, preferably enriched in favour of its (E)-isomer.

The compounds of formula (I) may be used alone, as mixtures thereof, or in combination with a base material. As used herein, the "base material" includes all known odorant molecules selected from the extensive range of natural products and synthetic molecules currently available, such as essential oils, alcohols, aldehydes and ketones, ethers and acetals, esters and lactones, macrocycles and heterocycles, and/or in admixture with one or more ingredients or excipients conventionally used in conjunction with odorants in fragrance compositions, for example, carrier materials, and other auxiliary agents commonly used in the art, e.g. solvents such as dipropylene glycol (DPG), isopropyl myristate (IPM), and thriethyl citrate (TEC).

The following list comprises examples of known odorant molecules, which may be combined with the compounds of the present invention:
- essential oils and extracts, e.g. tree moss absolute, basil oil, fruit oils such as bergamot oil and mandarine oil, myrtle oil, palmarose oil, patchouli oil, petitgrain oil, jasmine oil, rose oil, sandalwood oil, wormwood oil, lavender oil or ylang-ylang oil;
- alcohols, e.g. cinnamic alcohol, cis-3-hexenol, citronellol, Ebanol^{®}, eugenol, farnesol, geraniol, Super Muguet^{™}, linalool, menthol, nerol, phenylethyl alcohol, rhodinol, Sandalore^{®}, terpineol or Timberol^{®};
- aldehydes and ketones, e.g. anisaldehyde, α-amylcinnamaldehyde, Georgywood^{™}, hydroxycitronellal, Iso E^{®} Super, Isoraldeine^{®}, Hedione^{®}, Lilial^{®}, maltol, Methyl cedryl ketone, methylionone, verbenone or vanillin;
- ethers and acetals, e.g. Ambrox^{®}, geranyl methyl ether, rose oxide or Spirambrene;
- esters and lactones, e.g. benzyl acetate, Cedryl acetate, γ-decalactone, Helvetolide^{®}, γ-undecalactone or Vetivenyl acetate;
- macrocycles, e.g. Ambrettolide, Ethylene brassylate or Exaltolide^{®};
- heterocycles, e.g. isobutylquinoline.

The compounds according to formula (I) may be used in a broad range of fragrance applications, e.g. in any field of fine and functional perfumery, such as perfumes, household products, laundry products, body care products and cosmetics. The compounds can be employed in widely varying amounts, depending upon the specific application and on the nature and quantity of other odorant ingredients. The proportion is typically from 0.001 to 20 weight percent of the application. In one embodiment, compounds of the present invention may be employed in a fabric softener in an amount of from 0.001 to 0.05 weight percent. In another embodiment, compounds of the present invention may be used in fine perfumery in amounts of from 0.1 to 20 weight percent, more preferably between 0.1 and 5 weight percent. However, these values are given only by way of example, since the experienced perfumer may also achieve effects or may create novel accords with lower or higher concentrations.

The compounds as described hereinabove may be employed into the fragrance application simply by directly mixing the fragrance composition with the fragrance application, or they may, in an earlier step, be entrapped with an entrapment material, for example, polymers, capsules, microcapsules and nanocapsules, liposomes, film formers, absorbents such as carbon or zeolites, cyclic oligosaccharides and mixtures thereof, or they may be chemically bonded to substrates, which are adapted to release the fragrance molecule upon application of an external stimulus such as light, enzyme, or the like, and then mixed with the application.

Thus, the invention additionally provides a method of manufacturing a fragrance application, comprising the incorporation of a compound of the present invention, as a fragrance ingredient, either by directly admixing the compound to the application or by admixing a fragrance composition comprising a compound of formula (I), which may then be mixed to a fragrance application, using conventional techniques and methods.

As used herein, "fragrance application" means any product, such as fine perfumery, e.g. perfume and eau de toilette; household products, e.g. detergent for dishwasher, surface cleaner; laundry products, e.g. softener, bleach, detergent; body care products, e.g. shampoo, shower gel; and cosmetics, e.g. deodorant, vanishing creme, comprising an odorant. This list of products is given by way of illustration and is not to be regarded as being in any way limiting.

The α,β-unsaturated nitriles according to formula (I) may be conveniently prepared by Wittig reaction of saturated aldehydes with (triphenylphosphoranilydene)acetonitrile under conditions known to the skilled person.

The invention is now further described with reference to the following non-limiting examples. These examples are for the purpose of illustration only and it is understood that variations and modifications can be made by one skilled in the art.

### Example 1: Hex-2-enenitrile

A mixture of (triphenylphosphoranilydene)acetonitrile (5 g, 16.6 mmol), distilled butyraldehyde (1.2 g, 16.6 mmol), and distilled dibenzyl ether (15 ml) was heated 4 h at 80 °C. The reaction mixture (E/Z 70:30) was then cooled down and distilled using a Kugelrohr apparatus (ball-to-ball rotary distillation apparatus) at 40 mbar till 220 °C. The main fraction (1.59 g) was then microdistilled in the presence of 1.6 g paraffin (15 mbar, oil bath temperature: 120°C) giving a 63:37 mixture of (E/Z)-hex-2-enenitrile (0.6 g, 38%) having a boiling point of 79 °C at 15 mbar.

Odour description (E/Z)-hex-2-enenitrile: fruity, green, citrus.
Data of (E)-hex-2-enenitrile:
   ¹H-NMR (400MHz, CDCl₃): δ 6.72 *(dt,* J = 6.9, 16.4, H-C(3)), 5.33 (*dt,* J = 1.7, 16.4, H-C(2)), 2.21 (*qd*, J = 1.7, 7.2, H-C(4)), 1.49 *(sext.,* J = 7.3, H-C(5)), 0.94 (*t*, J = 7.3, *Me*CH₂).
   ¹³C-NMR (100MHz, CDCl₃): δ 155.91 (d, C(3)), 117.55 (s, CN), 99.78 (d, C(2)), 35.22 (*t*, C(4)), 20.90 (*t*, C(5)), 13.41 (*q*, C(6)).
   MS (EI): 95 (3), 94 (1), 80 (3), 68 (4), 67 (22), 66 (7), 65 (3), 64 (5), 63 (3), 62 (1), 55 (14), 54 (100), 53 (12), 52 (9), 51 (7), 50 (4), 43 (3), 42 (8), 41 (28), 40 (9), 39 (23), 38 (6), 29 (15), 28 (3), 27 (15).
   IR:νₘₐₓ 2964, 2935, 2876, 2223, 1633, 1461, 1437, 1382, 1341, 1309, 1053, 967, 795 cm⁻¹.
Data of (Z)-hex-2-enenitrile:
   ¹H-NMR (400MHz, CDCl₃): δ 6.49 (*dt*, J = 7.7, 10.9, H-C(3)), 5.32 (*dt*, J = 1.4, 10.9, H-C(2)), 2.41 (*qd,* J = 1.3, 7.4, H-C(4)), 1.50 (*sext.,* J = 7.3, H-C(5)), 0.97 (*t,* J = 7.3, *Me*CH₂).
   ¹³C-NMR (100MHz, CDCl₃): δ 154.97 (*d*, C(3)), 115.97 (s, CN), 99.63 (*d*, C(2)), 33.72 (*t*, C(4)), 21.52 (*t*, C(5)), 13.46 (*q*, C(6)).
   MS (EI): 95 (2), 94 (2), 80 (4), 68 (4), 67 (33), 66 (5), 65 (3), 64 (4), 63 (2), 62 (1), 55 (11), 54 (100), 53 (13), 52 (9), 51 (7), 50 (4), 43 (2), 42 (7), 41 (35), 40 (9), 39 (23), 38 (6), 29 (12), 28 (3), 27 (14).
   IR: νₘₐₓ 2964, 2935, 2875, 2220, 1621, 1461, 1381, 1338, 1241, 1210, 1075, 1044, 1003, 970, 916, 874, 852, 735 cm⁻¹.

### Example 2: Hept-2-enenitrile

Distilled valeraldehyde (1.43 g, 16.6 mmol) was added dropwise to a suspension of (triphenylphosphoranilydene)acetonitrile (5 g, 16.6 mmol) in distilled pentane (15 ml). After refluxing for 8 h, the reaction mixture was cooled down, filtered, and concentrated (rotary evaporator at 50 °C without vacuum). The residue (E:Z 70:30) was then distilled using a Kugelrohr apparatus (ball-to-ball rotary distillation apparatus) at 10 mbar and 81 °C giving two fractions of (E/Z)-hept-2-enenitrile (first fraction: 1.04 g, 58%, E:Z 64:36; second fraction: 0.45 g, E:Z 84:16, 25%) having a boiling point of 81 °C at 10 mbar.

Odour description: green, fruity, citrus, fatty.
Data of (E)-hept-2-enenitrile:
   ¹H-NMR (400MHz, CDCl₃): δ 6.73 (*dt*, J = 7.0, 16.3, H-C(3)), 5.33 (*dt*, J = 1.8, 16.3, H-C(2)), 2.23 (*qd*, J = 1.6, 7.2, H-C(4)), 1.51-1.29 (m, 4H), 0.92 *(t,* J = 7.3, *Me*CH₂).
   ¹³C-NMR (100MHz, CDCl₃): δ 156.14 (*d*, C(3)), 117.57 (s, CN), 99.59 (*d*, C(2)), 32.97 (*t*, C(4)), 29.66 (*t*, C(5)), 22.02 (*t*, C(5)), 13.67 (*q*, C(7)).
   MS (EI): 109 (1), 108 (2), 94 (5), 81 (4), 80 (5), 69 (10), 68 (11), 67 (32), 66 (7), 65 (3), 64 (4), 57 (4), 56 (100), 55 (10), 54 (54), 53 (14), 52 (10), 51 (8), 50 (4), 43 (41), 42 (10), 41 (73), 40 (7), 39 (28), 38 (4), 29 (5), 28 (5), 27 (20).
Data of (Z)-hept-2-enenitrile:
   ¹H-NMR (400MHz, CDCl₃): δ 6.49 (*dt,* J = 7.8, 10.9, H-C(3)), 5.31 (*dt*, J = 1.3, 10.9, H-C(2)), 2.43 (*qd,* J = 1.3, 7.3, H-C(4)), 1.51-1.29 (m, 4H), 0.93 (*t*, J = 6.8, MeCH₂).
   ¹³C-NMR (100MHz, CDCl₃): δ 155.23 (*d*, C(3)), 116.96 (*s*, CN), 99.40 (*d*, C(2)), 31.55 (*t*, C(4)), 30.26 (*t*, C(5)), 22.09 (*t*, C(5)), 13.70 (*q*, C(7)).
   MS (EI): 109 (1), 108 (3), 94 (7), 81 (6), 80 (7), 69 (12), 68 (13), 67 (76), 66 (6), 65 (4), 64 (4), 57 (4), 56 (100), 55 (9), 54 (55), 53 (18), 52 (13), 51 (10), 50 (5), 43 (49), 42 (12), 41 (82), 40 (8), 39 (30), 38 (5), 29 (6), 28 (6), 27 (23).

### Example 3: 4-Methylhex-2-enenitrile

A mixture of (triphenylphosphoranilydene)acetonitrile (5 g, 16.6 mmol), distilled 2-methylbutyraldehyde (1.43 g, 16.6 mmol), and distilled pentane (15 ml) was heated for 18 h at reflux, filtered, and concentrated (rotary evaporator at 50 °C without vacuum). FC (pentane) of the crude product (1.2 g) gave a 85:15 mixture of (E/Z)-4-methylhex-2-enenitrile (0.9 g, 50%) having a boiling point of 50 °C at 10 mbar.

Odour description: green, slightly cucumber, slightly fruity, slightly fatty-oily.
Data of (E)-4-methylhex-2-enenitrile:
   ¹H-NMR (400MHz, CDCl₃): δ 6.63 *(dd,* J = 7.8, 16.4, H-C(3)), 5.32 (*dd*, J = 1.3, 16.4, H-C(2)), 2.23 (br. *hept.t,* J = 1.1, 6.7, H-C(4)), 1.41 (*quint.,* J = 7.3, 2 H-C(5)), 1.05 (*d*, J = 6.7, MeC(4)), 0.89 (*t*, J = 7.4, MeC(5)).
   ¹³C-NMR (100MHz, CDCl₃): δ 161.03 (d, C(3)), 117.64 (s, CN), 98.25 (*d*, C(2)), 39.25 (*d*, C(4)), 28.42 (*t*, C(5)), 18.37 (*q*, MeC(4)), 11.37 (*q*, C(6)).
   MS (EI): 109 (1), 108 (1), 94 (3), 81 (8), 80 (20), 69 (6), 68 (25), 67 (13), 66 (7), 65 (4), 56 (100), 54 (22), 53 (24), 52 (10), 51 (8), 42 (12), 41 (22), 39 (14), 29 (7), 28 (3), 27 (10).
Data of (Z)-4-methylhex-2-enenitrile:
   ¹H-NMR (400MHz, CDCl₃): δ 6.25 (*t,* J = 10.6, H-C(3)), 5.28 (dd, J = 0.6, 10.6, H-C(2)), 2.75-2.62 (*m*, H-C(4)), 1.56-1.30 (*m*, 2 H-C(5)), 1.07 (d, J = 6.7, MeC(4)), 0.91 (*t*, J = 7.3, MeC(5)).
   ¹³C-NMR (100MHz, CDCl₃): δ 160.38 (*d*, C(3)), 118.41 (s, CN), 98.17 (*d*, C(2)), 38.56 (*d*, C(4)), 29.12 (*t*, C(5)), 19.56 (*q*, MeC(4)), 11.55 (*q*, C(6)).
   MS (EI): 109 (1), 108 (1), 94 (4), 81 (16), 80 (35), 69 (7), 68 (52), 67 (16), 66 (7), 65 (5), 56 (100), 54 (37), 53 (30), 52 (13), 51 (11), 42 (20), 41 (25), 39 (18), 29 (9), 28 (4), 27 (13).

### Example 4: 5-Methylhex-2-enenitrile

A mixture of (triphenylphosphoranilydene)acetonitrile (5 g, 16.6 mmol), distilled isovaleraldehyde (1.43 g, 16.6 mmol), and distilled pentane (15 ml) was heated for 6.5 h at reflux, filtered, and concentrated (rotary evaporator at 50 °C without vacuum). FC (pentane) of the crude product (2.1 g) gave a 66:34 mixture of (E/Z)-5-methylhex-2-enenitrile (1.0 g, 56%) having a boiling point of 64 °C at 10 mbar.

Odour description: green-fruity, fatty (isovalerianic).
Data of (E)-5-methylhex-2-enenitrile:
   ¹H-NMR (400MHz, CDCl₃): δ 6.70 (*dt,* J = 7.6, 16.3, H-C(3)), 5.33 *(dt,* J = 1.6, 16.3, H-C(2)), 2.12 (*ddd,* J = 1.5, 6.8, 7.5, 2 H-C(4)), 1.77 (*non.,* J = 6.7, H-C(5)), 0.93 (d, J = 6.7, 2 MeC(5)).
   ¹³C-NMR (100MHz, CDCl₃): δ 155.00 (*d*, C(3)), 117.46 (*s*, CN), 100.56 (*d*, C(2)), 42.44 (*t*, C(4)), 27.63 (*d*, C(5)), 22.10 (*q*, 2 MeC(5)).
   MS (EI): 109 (1), 108 (1), 94 (3), 81 (1), 68 (6), 67 (100), 66 (10), 65 (4), 56 (26), 53 (4), 52 (4), 51 (4), 43 (56), 41 (42), 39 (21), 27 (11).
Data of (Z)-5-methylhex-2-enenitrile:
   ¹H-NMR (400MHz, CDCl₃): δ 6.51 (*dt,* J = 7.8, 10.8, H-C(3)), 5.36 (*dt,* J = 1.4, 10.8, H-C(2)), 2.33 (*ddd,* J = 1.3, 6.8, 8.0, 2 H-C(4)), 1.80 (*non.,* J = 6.7, H-C(5)), 0.96 (*d*, J = 6.7, 2 MeC(5)).
   ¹³C-NMR (100MHz, CDCl₃): δ 154.00 (*d*, C(3)), 116.10 (*s*, CN), 100.18 (*d*, C(2)), 40.66 (*t*, C(4)), 27.98 (*d*, C(5)), 22.10 (*q*, 2 MeC(5)).
   MS (EI): 109 (1), 108 (1), 94 (2), 81 (1), 80 (1), 69 (1), 68 (5), 67 (100), 66 (6), 65 (3), 56 (6), 54 (2), 53 (3), 52 (3), 51 (3), 43 (24), 41 (28), 39 (15), 29 (1), 28 (1), 27 (7).

### Example 5: 4-Methylpent-2-enenitrile

A mixture of (triphenylphosphoranilydene)acetonitrile (5 g, 16.6 mmol), distilled isobutyraldehyde (1.2 g, 16.6 mmol), and distilled pentane (15 ml) was heated for 18 h at reflux, filtered, and concentrated (rotary evaporator at 50 °C without vacuum). The residue (E/Z 87:13) was then distilled using a Kugelrohr apparatus (ball-to-ball rotary distillation apparatus) at 10 mbar and 50 °C giving two fractions of (E/Z)-4-methylpent-2-enenitrile (first fraction: 0.16 g, 10%, E/Z 83:17; second fraction: 0.32 g, E/Z 91:9, 20%).
Boiling point: 50°C (10 mbar).

Odour description: green, fresh, fruity, slightly cuminic.
Data of (E)-4-methylpent-2-enenitrile:
   ¹H-NMR (400MHz, CDCl₃): δ 6.71 (*dd,* J = 6.6, 16.4, H-C(3)), 5.28 (*dd*, J =1.5, 16.4, H-C(2)), 2.48 (*oct*.*d*, J = 1.6, 6.8, H-C(4)), 1.07 (*d*, J = 6.8, 2 MeC(4)).
   ¹³C-NMR (100MHz, CDCl₃): δ 161.90 (*d*, C(3)), 117.69 (*s*, CN), 97.32 (*d*, C(2)), 32.05 (*d*, C(4)), 20.69 (*q*, 2 MeC(4)).
   MS (EI): 95 (13), 94 (37), 80 (59), 68 (71), 67 (48), 55 (52), 54 (28), 53 (100), 52 (28), 51 (20), 42 (21), 41 (26), 39 (28), 27 (16).
Data of (Z)-4-methylpent-2-enenitrile:
   ¹H-NMR (400MHz, CDCl₃): δ 6.31 (*dd*, J = 10.1, 10.9, H-C(3)), 5.21 *(dd,* J = 0.6, 10.9, H-C(2)), 2.92 (*dhept. d,* J = 0.5, 6.7, 10.1 H-C(4)), 1.09 (*d*, J = 6.6, 2 MeC(4)).
   ¹³C-NMR (100MHz, CDCl₃): δ 161.38 (*d*, C(3)), 117.69 (*s*, CN), 96.99 (*d*, C(2)), 31.57 (*d*, C(4)), 21.70 (*q*, 2 MeC(4)).
   MS (EI): 95 (12), 94 (40), 80 (60), 68 (76), 67 (48), 55 (52), 54 (30), 53 (100), 52 (29), 51 (20), 42 (23), 41 (29), 39 (31), 27 (17).

### Example 6: 3-Cyclopropylacrylonitrile

A mixture of (triphenylphosphoranilydene)acetonitrile (5 g, 16.6 mmol), distilled cyclopropanecarboxaldehyde (1.16 g, 16.6 mmol), and distilled pentane (15 ml) was heated for 19 h at reflux, filtered, and concentrated (rotary evaporator at 50 °C without vacuum). The residue was then distilled using a Kugelrohr apparatus (ball-to-ball rotary distillation apparatus) at 10 mbar and 67 °C giving two fractions of (E/Z)-3-cyclopropylacrylonitrile (first fraction: 0.27 g, 18%, E/Z 76:24; second fraction: 0.12 g, 8%, E/Z 85:15) having a boiling point of 67 °C at 10 mbar.

Odour description: green, apple, fruity, slightly fatty.
Data of (E)-3-cyclopropylacrylonitrile:
   ¹H-NMR (400MHz, CDCl₃): δ 6.13 (*dd*, J = 10.0, 16.0, H-C(3)), 5.36 (*d*, J = 16.0, H-C(2)), 1.64-1.55 (*m*, CH-C(3)), 1.04-0.98 (*m*, 2 H), 0.69-0.64 (*m*, 2 H).
   ¹³C-NMR (100MHz, CDCl₃): δ 159.94 (*d*, C(3)), 117.89 (*s*, CN), 95.88 (*d*, C(2)), 15.49 (*d*, CH-C(3)), 8.91 (*t,* 2 C).
   MS (EI): 94 (1), 93 (20), 78 (2), 67 (8), 66 (100), 65 (19), 64 (6), 63 (5), 54 (4), 53 (8), 52 (6), 51 (8), 50 (5), 41 (6), 40 (8), 39 (18), 38 (7), 37 (4), 27 (6).
Data of (Z)-3-cyclopropylacrylonitrile:
   ¹H-NMR (400MHz, CDCl₃): δ 5.80 *(dd,* J = 10.8, 16.0, H-C(3)), 5.18 *(dd,* J = 0.5, 10.8, H-C(2)), 2.06-1.95 (*m*, CH-C(3)), 1.10-1.05 (*m*, 2 H), 0.70-0.64 (*m*, 2 H).
   ¹³C-NMR (100MHz, CDCl₃): δ 159.25 (*d*, C(3)), 117.83 (*s*, CN), 95.65 (*d*, C(2)), 14.84 (*d*, CH-C(3)), 8.78 (*t,* 2 C).
   MS (EI): 94 (1), 93 (21), 78 (2), 67 (8), 66 (100), 65 (18), 64 (6), 63 (5), 54 (4), 53 (7), 52 (6), 51 (8), 50 (5), 41 (6), 40 (8), 39 (19), 38 (7), 37 (4), 27 (6).

### Example 7: A fragrance composition having a green apple accord

| Compound / Ingredient | parts by weight 1/1000 |
|---|---|
| Butyl Acetate | 20 |
| Geranyl Acetate | 10 |
| Hexenyl-3 cis Acetate | 3 |
| Hexyl Acetate | 50 |
| Nonanyl Acetate | 60 |
| Agrumex^{™} (2-tert-butylcyclohexyl acetate) | 140 |
| Cyclal C^{™} (2,4-dimethyl-3-cyclohexen-1-carbaldehyde) | 6 |
| Allyl Cyclo Hexyl Propionate (2-propenyl 3-cyclohexylpropanoate) | 8 |
| Delta Damascone | 2 |
| Gamma Decalactone | 7 |
| Dimyrcetol^{™} (mixture of 2,6-dimethyl-7octen-2-ol and 2,6-dimethyl-7-octen-2-yl formate) | 30 |
| Citronellyl Formate (3,7-dimethyloct-6-enyl formate) | 6 |
| Fructone^{™} (ethyl methyl dioxolane acetate) | 50 |
| Geraniol | 40 |
| Givescone^{®} (mixture of ethyl 2-ethyl-6,6-dimethyl-2-cyclohexenecarboxylate & ethyl 2,3,6,6-tetramethyl-2-cyclohexenecarboxylate) | 5 |
| lonone Beta | 20 |
| Labienoxime (2,4,4,7-tetramethyl-6,8-nonadiene-3-one oxime) 1 % solution in mixture of isopropyl myristate and triethyl citrate | 3 |
| Linalool (3,7-dimethyl-1,6-octadien-3-ol) | 30 |
| Diethyl Malonate | 160 |
| Ethyl 2-methylbutyrate | 10 |
| Isopropyl 2-methylbutyrate | 10 |
| Pharaone (2-cyclohexyl-1,6-heptadien-3-one) 10% in DPG | 10 |
| Dipropylene Glycol (DPG) | 300 |
| Hex-2-enenitrile | **20** |

In this apple accord 2% of hex-2-enenitrile provides a typical green apple volume and makes it more fresh-natural and tasty.

## Claims

1. Use as flavour or fragrance ingredient of a compound of formula (I) 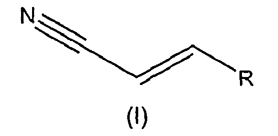 wherein R is selected from linear, branched C₃₋₄ alkyl and C₃₋₄ cycloalkyl; and the double bond is in E or Z configuration;
or a mixture thereof.

2. The use according to claim 1 wherein the compound of formula (I) is selected from the group consisting of hex-2-enenitrile, hept-2-enenitrile, 4-methylhex-2-enenitrile, 5-methylhex-2-enenitrile, 4-methylpent-2-enenitrile and 3-cyclopropylacrylonitrile.

3. A fragrance composition comprising a compound of formula (I) 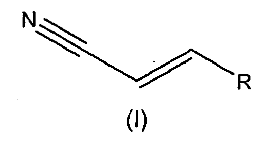 wherein R is selected from linear, branched C₃₋₄ alkyl and C₃₋₄ cycloalkyl; and the double bond is in E or Z configuration;
or a mixture thereof; and a base material.

4. A method of manufacturing a fragrance composition, comprising incorporating a compound of formula (I) 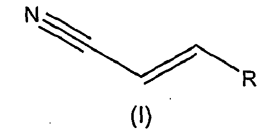 wherein R is selected from linear, branched C₃₋₄ alkyl and C₃₋₄ cycloalkyl; and the double bond is in E or Z configuration;
or a mixture thereof;
into a base material.

5. A method of improving, enhancing or modifying a fragrance application through the addition of an olfactory acceptable amount of a compound of formula (I) 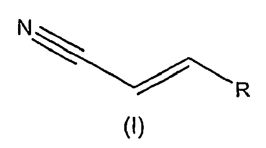 wherein R is selected from linear, branched C₃₋₄ alkyl and C₃₋₄ cycloalkyl; and the double bond is in E or Z configuration;
or a mixture thereof.

6. A method according to claim 4 or claim 5 wherein the fragrance application is selected from the group consisting of perfumes, household products, laundry products, body care products and cosmetics.

7. A compound of formula (I) 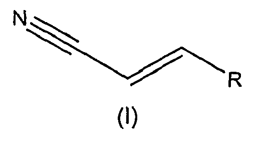 wherein R is 1-methylprop-1-yl; and the double bond is in E or Z configuration; or a mixture thereof.

## Patentansprüche

1. Verwendung einer Verbindung der Formel (I) 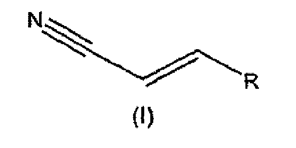 worin R unter linearem, verzweigtem C₃₋₄-Alkyl und C₃₋₄-Cycloalkyl ausgewählt ist und
die Doppelbindung in E- oder Z-Konfiguration vorliegt;
oder einer Mischung davon als Geschmacksstoff- oder Duftstoffbestandteil.

2. Verwendung nach Anspruch 1, bei der die Verbindung der Formel (I) aus der Gruppe bestehend aus Hex-2-ennitril, Hept-2-ennitril, 4-Methylhex-2-ennitril, 5-Methylhex-2-ennitril, 4-Methylpent-2-ennitril und 3-Cyclopropylacrylnitril ausgewählt ist.

3. Duftstoffzusammensetzung, umfassend eine Verbindung der Formel (I) 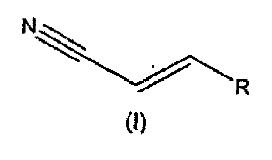 worin R unter linearem, verzweigtem C₃₋₄-Alkyl und C₃₋₄-Cycloalkyl ausgewählt ist und
die Doppelbindung in E- oder Z-Konfiguration vorliegt;
oder eine Mischung davon und einen Grundstoff.

4. Verfahren zur Herstellung einer Duftstoffzusammensetzung, bei dem man eine Verbindung der Formel (I) 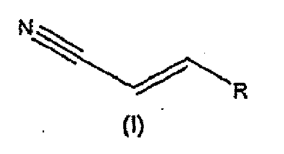 worin R unter linearem, verzweigtem C₃₋₄-Alkyl und C₃₋₄-Cycloalkyl ausgewählt ist und
die Doppelbindung in E- oder Z-Konfiguration vorliegt;
oder eine Mischung davon
in einen Grundstoff einarbeitet.

5. Verfahren zur Verbesserung, Verstärkung oder Modifizierung einer Duftstoffanwendung durch Zugabe einer olfaktorisch annehmbaren Menge einer Verbindung der Formel (I) 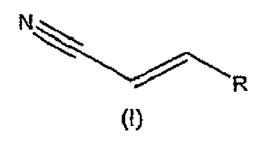 worin R unter linearem, verzweigtem C₃₋₄-Alkyl und C₃₋₄-Cycloalkyl ausgewählt ist und
die Doppelbindung in E- oder Z-Konfiguration vorliegt;
oder einer Mischung davon.

6. Verfahren nach Anspruch 4 oder Anspruch 5, bei dem man die Duftstoffanwendung aus der Gruppe bestehend aus Parfümen, Haushaltsprodukten, Waschmittelprodukten, Körperpflegeprodukten und Kosmetika auswählt.

7. Verbindung der Formel (I) 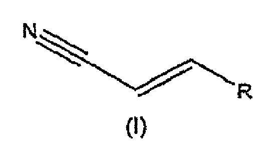 worin R für 1-Methylprop-1-yl steht und die Doppelbindung in E- oder Z-Konfiguration vorliegt;
oder eine Mischung davon.

## Revendications

1. Utilisation en tant qu'ingrédient d'arôme ou de parfum d'un composé de formule (I) 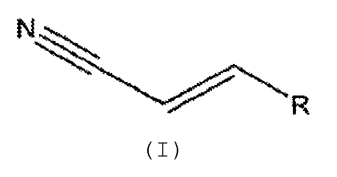 dans laquelle R est choisi parmi un alkyle en C₃₋₄ linéaire ou ramifié et un cycloalkyle en C₃₋₄ ; et
la double liaison est dans la configuration E ou Z ;
ou un mélange de celui-ci.

2. Utilisation selon la revendication 1 **caractérisée en ce que** le composé de formule (I) est choisi dans le groupe constitué de l'hex-2-ènenitrile, l'hept-2-ènenitrile, le 4-méthylhex-2-ènenitrile, le 5-méthylhex-2-ènenitrile, le 4-méthylpent-2-ènenitrile et le 3-cyclopropylacrylonitrile.

3. Composition de parfum comprenant un composé de formule (I) 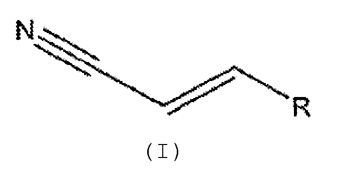 dans laquelle R est choisi parmi un alkyle en C₃₋₄ linéaire ou ramifié et un cycloalkyle en C₃₋₄ ; et
la double liaison est dans la configuration E ou Z ;
ou un mélange de celui-ci ; et un matériau de base.

4. Procédé de fabrication d'une composition de parfum, comprenant l'incorporation d'un composé de formule (I) 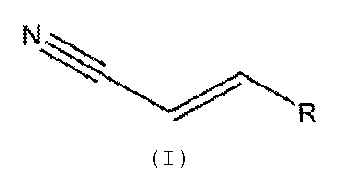 dans laquelle R est choisi parmi un alkyle en C₃₋₄ linéaire ou ramifié et un cycloalkyle en C₃₋₄ ; et
la double liaison est dans la configuration E ou Z ;
ou un mélange de celui-ci ;
dans un matériau de base.

5. Procédé d'amélioration, de renforcement ou de modification d'une application de parfum par ajout d'une quantité acceptable sur le plan olfactif d'un composé de formule (I) 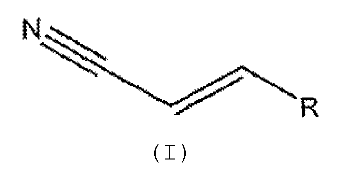 dans laquelle R est choisi parmi un alkyle en C₃₋₄ linéaire ou ramifié et un cycloalkyle en C₃₋₄ ; et
la double liaison est dans la configuration E ou Z ;
ou un mélange de celui-ci.

6. Procédé selon la revendication 4 ou la revendication 5 **caractérisé en ce que** l'application de parfum est choisie dans le groupe constitué de parfums, produits domestiques, produits de blanchisserie, produits de soins corporels et cosmétiques.

7. Composé de formule (I) 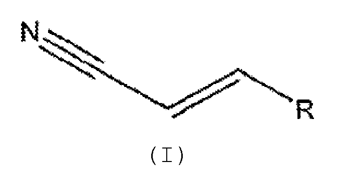 **caractérisé en ce que** R est le 1-méthylprop-1-yle ; et la double liaison est dans la configuration E ou Z ; ou un mélange de celui-ci.
